# EUROPÄISCHE PATENTSCHRIFT

(11) **EP 0 895 982 B1**
(45) Veröffentlichungstag und Bekanntmachung des Hinweises auf die Patenterteilung: **12.12.2001**
(21) Anmeldenummer: 98112986.9
(22) Anmeldetag: 13.07.1998
(51) Int. Cl.: C07C 67/54, C07C 69/675, B01D 3/00

(54) **Verfahren zur Gewinnung von Hydroxypivalinsäureneopentyl-glykolester (HPN)**
Process for the purification of hydroxypivalyl hydroxypivalate
Procédé de purification de l'hydroxypivalate d'hydroxypivalyle

(30) Priorität: 05.08.1997 DE 19733903
(43) Veröffentlichungstag der Anmeldung: 10.02.1999
(73) Patentinhaber: BASF AKTIENGESELLSCHAFT, 67056 Ludwigshafen (DE)
(72) Erfinder: Auer, Heinz, 68809 Neulussheim (DE); Krüger, Siegfried, 67346 Speyer (DE); Scholl, Stephan, Dr., 67098 Bad Dürkheim (DE); Weber, Theodor, 67063 Ludwigshafen (DE); Melder, Johann-Peter, Dr., 67141 Neuhofen (DE)
(74) Vertreter: Isenbruck, Günter, Dr.

(56) Entgegenhaltungen:
- WO-A-92/07815
- DE-A- 2 234 358

## Beschreibung

Die Erfindung betrifft ein Verfahren zur Gewinnung von Hydroxypivalinsäureneopentylglykolester (HPN) aus einem HPN, leichter- und höhersiedende Produkte und anorganische Salze enthaltenden Gemisch. Die Erfindung betrifft zudem eine Vorrichtung zur Ausführung des Verfahrens.

Hydroxypivalinsäureneopentylglykolester (HPN), auch als Hydroxypivalylhydroxypivalat bezeichnet, wird technisch beispielsweise mittels der Tishchenko-Reaktion aus Hydroxypivalaldehyd in Gegenwart von basischen anorganischen Katalysatoren hergestellt. Hydroxypivalaldehyd wird dabei beispielsweise aus Isobutyraldehyd und Formaldehyd hergestellt. Das so hergestellte HPN kann größere Mengen an Verunreinigungen, wie Isobutyraldehyd, Isobutanol, nicht ungesetzten Hydroxypivalaldehyd, Neopentylglykol und Neopentylglykolmonoisobutyrat neben hochsiedenden Produkten und anorganischen Katalysatorresten enthalten. Ein Teil der Verunreinigungen kann schon durch den in der Umsetzung eingesetzten Hydroxypivalaldehyd eingebracht werden.

HPN ist eine thermisch empfindliche Substanz, die beim Aufheizen auf höhere Temperaturen, insbesondere über 150°C bei längeren Verweilzeiten beginnt, sich zu zersetzen. Aus diesem Grund ist die Reinigung von HPN durch thermische Verfahren, wie Destillationen schwierig. Es muß versucht werden, die thermische Belastung möglichst gering zu halten.

Unterschiedliche Verfahren zur Reinigung dieses Roh-HPN-Gemisches sind beschrieben.

In den US 3,641,118 und US 3,696,005 sind Verfahren zur Reinigung von HPN mit Hilfe von Säuren bzw. Säurenionentauschern beschrieben. Beispielsweise wird eine Destillation in Gegenwart einer Säure durchgeführt.

Zur Abtrennung von Salzen, wie Calciumhydroxid, Bariumhydroxid oder Strontiumhydroxid ist in der US 4,665,219 vorgeschlagen worden, den Wassergehalt des Reaktionsgemisches auf 20 bis 60% einzustellen und das Salz bei einer Temperatur von 50 bis 100°C zu extrahieren.

In der EP-A-0 410 167 ist ein Verfahren zur Herstellung von HPN und eutektischen Gemischen aus Neopentylglykol und HPN aus dem Nebenproduktstrom aus einer Neopentylglykolherstellung beschrieben. Das Umsetzungsprodukt kann mit Hilfe eines Wischfilmverdampfers und einer Reihe von Kondensatoren destilliert werden, wobei HPN durch ein weiteres, nicht genauer spezifiziertes Destillationsverfahren weiter abgetrennt werden kann.

In der US 4,935,555 ist ein Verfahren zur Herstellung von Neopentylglykol beschrieben, wobei das Rohprodukt mit Natronlauge versetzt und mit einem Wischfilmverdampfer gereinigt wird. Der Wischfilmverdampfer kann dabei mit einer Kolonne verbunden sein, an deren Kopf Leichtsieder, aus deren Sumpf Hochsieder und aus deren Seitenaustrag Neopentylglykol entnommen werden. Der Sumpf aus dem Wischfilmverdampfer wird dabei einer Dekantiervorrichtung zugeführt.

In der EP-B-0 555 335 ist ein Verfahren zur Gewinnung von Hydroxypivalylhydroxypivalat aus einer Rohmischung aus der Synthese beschrieben, bei dem das Gemisch in einer ersten Destillationseinheit einem Wischfilmverdampfer zugeführt wird, in dem anorganische Salze und hochsiedende Bestandteile als Sumpf entfernt werden. Der dampfförmige Austrag wird einer Teilkondensatsäule zugeführt, in der hochsiedende Bestandteile kondensiert werden. Die aus der Teilkondensatsäule gewonnenen Dämpfe von HPN und leichtsiedenden Produkten werden einer Leichtsiedersäule in einer zweiten Destillationseinheit zugeführt. In der Leichtsiedersäule werden über Kopf die leichtsiedenden Produkte entnommen und kondensiert, wobei ein Teil des Kondensats an den Kopf der Kolonne zurückgeführt werden kann. Der Sumpfaustrag aus der Kolonne wird einem zweiten Wischfilmverdampfer zugeführt, aus dessen Dampfaustrag HPN ausgeschleust wird. Ein verbleibender Dampfanteil kann in die Kolonne zurückgeführt werden. Aus dem Sumpfaustrag des zweiten Wischfilmverdampfers werden hochsiedende Produkte ausgeschleust. Diese können als Rückstand abgezogen oder in den ersten Wischfilmverdampfer zurückgeführt werden. Ein Nachteil dieses Verfahrens liegt in der notwendigen Verwendung zweier Wischfilmverdampfer, die sehr kostenaufwendig sind. Zudem ist die erste Destillationseinheit mit der zweiten Destillationseinheit druckgekoppelt und kann nicht unter unabhängigen Verfahrensbedingungen betrieben werden. Dadurch ist das Verfahren schwierig zu steuern und empfindlich gegen Störungen und Schwankungen. Zudem werden in der Regel zwei Rückstandsströme aus den beiden Wischfilmverdampfern erzeugt.

Ein weiterer Nachteil besteht darin, daß das Rein-HPN nicht vollständig von hochsiedenden, aber nahe dem Siedepunkt von HPN siedenden, Verunreinigungen befreit werden kann, da zwischen Hochsiederausschleusung am zweiten Wischfilmverdampfer und Rein-HPN-Abzug nur eine einstufige Abdampfung und keine Rektifikation vorliegt.

Aufgabe der vorliegenden Erfindung ist die Bereitstellung eines Verfahrens zur Gewinnung von HPN aus einem HPN, leichter- und höhersiedende Produkte und anorganische Salze enthaltenden Gemisch, das die Herstellung von sehr reinem HPN in hoher Ausbeute und ohne farbige Verunreinigungen erlaubt, wobei das Verfahren weniger aufwendig als die bekannten Verfahren ist, eine gute Steuerung der einzelnen Verfahrensstufen zuläßt und unanfällig gegenüber Störungen ist.

Erfindungsgemäß wird die Aufgabe gelöst durch ein Verfahren zur Gewinnung von Hydroxypivalinsäureneopentylglykolester (HPN) aus einem HPN, leichter- und höhersiedende Produkte und anorganische Salze enthaltenden Gemisch, bei dem das Gemisch in einer ersten Trennstufe aus einem Wischfilmverdampfer und einem Wärmeübertrager
- dem Wischfilmverdampfer zugeführt wird,
- in dem als Sumpf ein Strom aus höhersiedenden Produkten und anorganischen Salzen ausgetragen wird
- und in dem über Kopf ein dampfförmiger Strom aus HPN, leichter- und höhersiedenden Produkten ausgetragen, dem Wärmeübertrager zugeführt und in ihm kondensiert wird, und der erhaltene Kondensatstrom
in einer zweiten Trennstufe aus einer Rektifizierkolonne zur Trennung von HPN, leichter- und höhersiedenden Produkten, einem zweiten Verdampfer und Wärmeübertragern
- der Kolonne zugeführt wird,
- in der über Kopf ein dampfförmiger Strom aus leichtsiedenden Produkten ausgetragen und in einem Wärmeübertrager kondensiert wird,
- das erhaltene Kondensat gegebenenfalls teilweise als Rücklauf auf die Rektifizierkolonne zurückgeführt und teilweise als Leichtsiederausschleusung abgezogen wird,
- in einem Seitenabzug ein dampfförmiger Strom aus HPN ausgetragen und in einem Wärmeübertrager kondensiert wird
- und als Sumpf ein Strom aus HPN und höhersiedenden Produkten ausgetragen wird, der zumindest teilweise dem zweiten Verdampfer zugeführt wird, wobei der Verdampferaustrag ganz oder teilweise in den unteren Bereich der Kolonne zurückgeführt wird.

Erfindungsgemäß wurde gefunden, daß durch Verwendung eines Wärmeübertragers zur vollständigen Kondensation des Dampfauslasses aus dem Wischfilmverdampfer der ersten Trennstufe und Zuführung des flüssigen Kondensats in die zweite Trennstufe eine Druckentkoppelung der beiden Stufen möglich ist. Damit können in den beiden Trennstufen die Drücke frei gewählt werden und somit die Verfahrensbedingungen flexibler eingestellt werden. Störungen und Prozeßschwankungen, wie Druckschwankungen, wirken sich nur auf die entsprechende Trennstufe, nicht jedoch auf das gesamte Verfahren aus. Bei Störungen können die beiden Trennstufen unabhängig voneinander außer Betrieb genommen werden.

Zudem muß im Verfahren gemäß EP-B-0 555 335 in der Teilkondensatsäule eine ganze Anzahl von Parametern exakt eingehalten werden, um eine Abtrennung von hochsiedenden Produkten an dieser Stelle sicher zu gewährleisten. So muß die Temperatur im Bereich von 150 bis 175°C liegen, ein Druck von 5 bis 30 Torr herrschen und gleichzeitig am Säulenkopf eine um 1 bis 5°C niedrigere Temperatur und ein um 1 bis 5 Torr niedrigerer Druck als an der Säulenbasis herrschen. Nur unter diesen Bedingungen ist sichergestellt, daß keine höhersiedenden Produkte in die zweite Destillationszone gelangen, wo ihre Abtrennung nicht mehr möglich ist, da bei der zweiten Wischfilmverdampfung nur noch eine einstufige Abdampfung erfolgt. Besonders kritisch sind dabei höhersiedende Verunreinigungen mit einem Siedepunkt höher aber nahe am Siedepunkt von Rein-HPN, wie alle Kondensationsprodukte. Diese können effektiv nur unter Ausnutzung des Rektifiziereffektes gegen Rein-HPN getrennt werden.

Erfindungsgemäß erfolgt die Abtrennung von hochsiedenden Produkten im ersten Wischfilmverdampfer in der ersten Trennstufe, wie auch in der Rektifizierkolonne in der zweiten Trennstufe. Hierdurch ist es möglich, nach dem ersten Wischfilmverdampfer einen Wärmeübertrager zur vollständigen Kondensation zu betreiben, in dem die Verfahrensbedingungen unkritisch sind.

Zudem kann im erfindungsgemäßen Verfahren als zweiter Verdampfer in der zweiten Trennstufe ein beliebiger Verdampfer eingesetzt werden, der eine Verdampfung des Sumpfaustrags mit geringer thermischer Belastung erlaubt. Der Verdampfer dient zur Brüdenerzeugung für die Rektifizierkolonne, d.h. zur Bereitstellung der für die Rektifikation des zu trennenden Stoffgemisches in der Kolonne erforderlichen Brüdenstromes. Eine Reinigung von HPN erfolgt in der Kolonne durch Entnahme an einem Seitenabzug der Kolonne oberhalb der ersten Trennsektion. Gemäß EP-B-0 555 335 erfolgt die Reinigung und Abtrennung von HPN durch den zweiten Wischfilmverdampfer, der somit neben der Brüdenerzeugung auch der Reinigung von HPN durch Verdampfung und der Ausschleusung eines Rückstandsstroms von hochsiedenden Produkten dient.

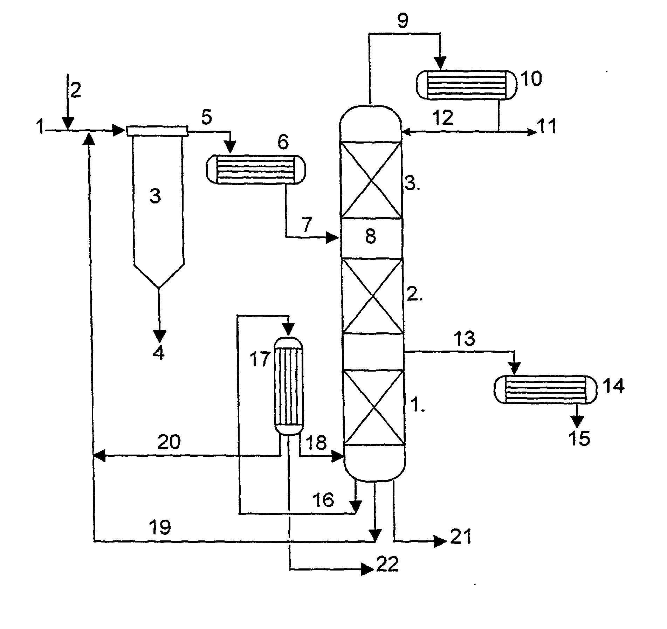

Nachstehend werden bevorzugte Ausführungsformen der Erfindung anhand der Zeichnung erläutert, die
in Figur 1 eine schematische Darstellung des erfindungsgemäßen Verfahrens zeigt.

Dabei bedeuten:
- 1.:: 1. Trennsektion
- 2.:: 2. Trennsektion
- 3.:: 3. Trennsektion

- (1):: Zuführung Roh-HPN-Gemisch
- (2):: Zuführung Fließmittel
- (3):: Wischfilmverdampfer der ersten Trennstufe
- (4):: Sumpfauslaß des Wischfilmverdampfers (3)
- (5):: Zuführung vom Dampfauslaß des Wischfilmverdampfers (3) zum Wärmeübertrager (6)
- (6):: Wärmeübertrager
- (7):: Zuführung vom Austritt des Wärmeübertragers (6) zur Kolonne (8)
- (8):: Rektifizierkolonne
- (9):: Zuführung vom Kopf der Kolonne (8) zum Wärmeübertrager (10)
- (10):: Wärmeübertrager
- (11):: Abzug Kopfprodukt aus dem Wärmeübertrager (10)
- (12):: Rücklauf der Kolonne (8)
- (13):: Seitenabzug der Kolonne (8)
- (14):: Wärmeübertrager
- (15):: Auslaß aus dem Wärmeübertrager (14)
- (16):: Umlaufstrom zum Verdampfer (17)
- (17):: Verdampfer der zweiten Trennstufe
- (18):: Zuführung vom Ablauf des Verdampfers (17) zum Sumpfbereich der Kolonne (8)
- (19):: Zuführung aus dem Sumpf der Kolonne (8) zum Zulauf des Wischfilmverdampfers (3) der ersten Trennstufe
- (20):: Zuführung aus dem Ablauf des Verdampfers (17) zum Zulauf des Wischfilmverdampfers (3) der ersten Trennstufe
- (21):: Auslaß aus dem Sumpf der Kolonne (8)
- (22):: Auslaß aus dem Ablauf des Verdampfers (17)

Das dem Wischfilmverdampfer (3) zugeführte Gemisch (1) aus HPN, leichter- und höhersiedenden Produkten und anorganischen Salzen enthält vorzugsweise 75 bis 85 Gew.-% HPN, 15 bis 25 Gew.-% leichtersiedende Produkte, 0,1 bis 2,0 Gew.-% höhersiedende Produkte und 0,1 bis 2,0 Gew.-% anorganische Salze, wobei die Summe der Bestandteile insgesamt 100 Gew.-% ergibt. Leichtersiedende Produkte sind solche, deren Siedepunkt niedriger als der Siedepunkt von HPN (160°C bei 10,5 mbar) ist. Beispiele solcher Produkte sind Hydroxypivalinsäure (HPA), Isobutyraldehyd, Isobutanol, Formaldehyd, Hydroxypivalaldehyd (HPA), Neopentylglykol, Neopentylglykolmonoisobutyrat und Neopentylglykolmonoformiat.

Höhersiedende Produkte sind solche Produkte, die einen höheren Siedepunkt als HPN aufweisen. Beispiele hierfür sind höhere Kondensationsprodukte von HPN mit Hydroxypivalinsäure oder Isobuttersäure und isomere Kondensationsprodukte von HPN mit Ameisensäure.

Anorganische Salze sind vornehmlich die bei der Herstellung von HPN eingesetzten anorganischen basischen Katalysatoren, insbesondere Erdalkalimetallhydroxide, wie Calciumhydroxid, Bariumhydroxid oder Strontiumhydroxid.

Gemäß einer bevorzugten Ausführungsform der Erfindung wird dem vorstehend beschriebenen (Roh-HPN)-Gemisch eine hochsiedende und unter den Verarbeitungsbedingungen und im Hinblick auf die Verarbeitungsprodukte inerte Verbindung als Fließmittel (2) zugesetzt. Dieses Fließmittel dient dazu, ein Anhaften von im ersten Wischfilmverdampfer ausfallenden anorganischen Salzen zu verhindern. Das Fließmittel weist daher einen so hohen Siedepunkt auf, daß es im Wischfilmverdampfer nicht verdampft wird, sondern als Sumpf anfällt. Geeignete Fließmittel sind Polyether (mit alkylierten Endgruppen -OR) und Polyetherdiole (mit Hydroxylendgruppen), besonders bevorzugt Polyethylenglykol mit einem mittleren Molekulargewicht von 400 bis 600 g/mol. Das Fließmittel wird vorzugsweise in einer Menge von 0,2 bis 3,0 Gew.-%, bezogen auf den Zulauf (1) als 100 Gew.-%, eingesetzt. Besonders bevorzugte Mengen liegen im Bereich von 0,2 bis 1,0 Gew.-%. Durch Verwendung des Fließmittels kann im Wischfilmverdampfer eine sehr weitgehende Verdampfung der verdampfbaren Verbindungen des eingesetzten Roh-HPN-Gemisches erreicht werden, ohne daß Wärmeübertragerflächen des Wischfilmverdampfers unbenetzt bleiben. Die Verdampfungseffektivität kann dadurch erhöht und die thermische Belastung des eingesetzten HPN verringert werden, so daß ein thermischer Abbau von HPN im Verdampfer vermieden wird.

Als Wischfilmverdampfer kann jeder geeignete Wischfilmverdampfer, in dem der Film mechanisch durch ein Wischsystem mit Wischerblättern bewegt wird, verwendet werden. Das Wischersystem kann dabei starre oder bewegliche Wischerblätter aufweisen. Besonders bevorzugt wird ein Wischfilmverdampfer mit beweglichen Wischerblättern eingesetzt. Der Verdampfer der ersten Trennstufe wird vorzugsweise bei einem Druck von 2 bis 15, besonders bevorzugt 5 bis 10 mbar, einer Manteltemperatur von vorzugsweise 140 bis 200°C, besonders bevorzugt 160 bis 180°C betrieben. Als Sumpfaustrag werden anorganische Salze und hochsiedende Produkte abgetrennt. Bei Verwendung von Fließmitteln werden diese ebenfalls als Sumpfaustrag (3) abgetrennt.

Der dampfförmige Austrag (5) des Wischfilmverdampfers wird einem Wärmeübertrager (6) zugeführt, in dem eine Kondensation, vorzugsweise eine möglichst vollständige Kondensation des Dampfaustrags stattfindet. Als Wärmeübertrager sind dabei alle technisch einsetzbaren Bauformen für Kondensatoren geeignet. Der Druck im Wärmeübertrager beträgt vorzugsweise 2 bis 15, besonders 5 bis 10 mbar, die Temperatur vorzugsweise 30 bis 130, besonders bevorzugt 55 bis 110°C. Der Dampfaustrag (5) enthält dabei überwiegend HPN und niedrig- und hochsiedende Produkte, die vollständig oder nahezu vollständig kondensiert der Kolonne (8) zugeführt werden.

Die Kolonne ist mit geeigneten Einbauten zur Trennung von HPN, leicht- und höhersiedenden Produkten ausgerüstet. Besonders geeignet sind druckverlustarme Einbauten in den Trennsektionen, vorzugsweise strukturierte oder geordnete Packungen. Die Kolonne und ihre Einbauten sind dabei vorzugsweise so ausgeführt, daß am Kopf der Kolonne ein dampfförmiger Strom aus leichtsiedenden Produkten, in einem Seitenabzug ein dampfförmiger Strom aus HPN und als Sumpf ein Strom aus HPN und höhersiedenden Produkten ausgetragen wird. Vorzugsweise wird die Rektifizierkolonne mit mindestens drei Trennsektionen ausgeführt, siehe Fig. 1. Der Seitenabzug (13) wird zwischen der ersten und zweiten Trennsektion entnommen, der Zulauf (7) aus dem Wärmeübertrager (6) erfolgt zwischen der zweiten und dritten Trennsektion.

Durch die spezifische Kombination von Trennsektionen ist es möglich, HPN in sehr hoher Reinheit zu erhalten. Eine Trennung auch gegenüber den nächst höhersiedenden Verunreinigungen wird erreicht. Diese hohe Produktreinheit kann auch bei Störungen oder Schwankungen, beispielsweise in der Zusammensetzung des Roh-HPN und damit des eingespeisten Kondensats aus der ersten Trennstufe oder bei Änderungen der Einspeisungsmenge, zuverlässig erreicht werden.

Vorzugsweise weist die Kolonne 6 bis 20, besonders bevorzugt 10 bis 14 theoretische Böden auf. Dabei weist die erste Trennsektion vorzugsweise 2 bis 6, insbesondere 3 bis 5 theoretische Böden, die zweite Trennsektion vorzugsweise 3 bis 9, besonders bevorzugt 4 bis 7 theoretische Böden und die dritte Trennsektion vorzugsweise 2 bis 6, besonders bevorzugt 3 bis 5 theoretische Böden auf. Die Kolonne kann dabei auch weitere Trennsektionen aufweisen. Vorzugsweise werden drei Trennsektionen verwendet. Der Kopfdruck der Kolonne beträgt 3 bis 20 mbar, bevorzugt 4 bis 15 mbar, besonders bevorzugt 5 bis 10 mbar. Die Temperatur der Kolonne beträgt dabei im Sumpf vorzugsweise 150 bis 180, besonders bevorzugt etwa 165 bis 175°C, zwischen der ersten und zweiten Trennsektion und/oder der zweiten und dritten Trennsektion vorzugsweise 150 bis 170, besonders bevorzugt 155 bis 165°C und am Kolonnenkopf vorzugsweise 90 bis 100, besonders bevorzugt 93 bis 97°C. Die Temperatur des Wärmeübertragers zur Kondensierung von HPN am Seitenabzug beträgt vorzugsweise 30 bis 130, besonders bevorzugt 55 bis 110°C.

Der am Kopf der Kolonne gewonnene und kondensierte Austrag an leichtsiedenden Produkten kann aus dem Verfahren ausgeschleust und weiterverwendet oder entsorgt werden. Bei einem hohen Anteil an HPA oder anderen Edukten aus der Synthese von HPN können diese, gegebenenfalls nach weiterer Aufreinigung, wieder in die Synthese zurückgeführt werden. Ein Teil des Kondensatstroms oder der gesamte Kondensatstrom kann auch in den Kopf der Kolonne zurückgeführt werden. Vorzugsweise beträgt das Rücklaufverhältnis, d.h. das Verhältnis von zurückgeführtem Kondensatstrom (12) zu ausgeschleustem Kondensatstrom (11) mehr als 5, besonders bevorzugt 5 bis 25, insbesondere 10 bis 20.

In der ersten Trennsektion werden höhersiedende Verunreinigungen im HPN aufkonzentriert. Dem Sumpf der Kolonne (8) wird zum einen der Umlaufstrom (16) zum Verdampfer (17) entnommen, zum anderen die Hochsiederausschleusung. Letztere wird vorzugsweise als Hochsiederrückführung (19) zum Zulauf des Wischfilmverdampfers (3) zurückgeführt. Optional kann sie auch als Rückstandsstrom (21) ausgeschleust werden. Alternativ zu Strom (19) kann der hochsiederreiche Rückführstrom (20) auch dem nicht verdampften Flüssigkeitsstrom aus dem Verdampfer (17) entnommen werden. Dies entspricht der Realisierung eines getrennten Sumpfes, wie er in DE-A-33 38 488 beschrieben ist. Analog zum Strom (21) kann die Hochsiederausschleusung als Strom (22) aus dem nicht verdampften Flüssigkeitsablauf des Verdampfers (17) erfolgen. Die Ströme (4), (21) und (22) können entsorgt oder einer weiteren Aufarbeitung zugeführt werden.

Das aus dem Seitenabzug gewonnene Rein-HPN (15) weist vorzugsweise einen HPN-Gehalt von mehr als 98 Gew.-%, eine Hazen-Farbzahl von weniger als 10 APHA, einen Wassergehalt von weniger als 0,3 Gew.-% und eine Säurezahl von weniger als 5 auf. Aus dem Flüssigkeitsablauf des Verdampfers der zweiten Trennstufe kann ein Strom in den Wischfilmverdampfer der ersten Trennstufe zurückgeführt werden.

Aus dem Flüssigkeitsablauf des Verdampfers der zweiten Trennstufe kann ein Strom ausgeschleust werden.

Bei der Durchführung des erfindungsgemäßen Verfahrens werden vorzugsweise die nachstehenden Mengenströme eingesetzt beziehungsweise geregelt:

Setzt man den Roh-HPN-Zulauf (1) zu 100 Gew.-% an, so beträgt der Zulaufstrom (2) mit Fließmittel 0,5 bis 4 Gew.-%, bevorzugt 1 bis 2 Gew.-%. Der Sumpfaustrag (4) des Wischfilmverdampfers (3) beträgt 1 bis 10 Gew.-%, bevorzugt 2 bis 8 Gew.-%, besonders bevorzugt 2 bis 6 Gew.-%. Das Verhältnis von Rückführstrom zu Frischzulauf (19)/(1) bzw. (20)/(1) beträgt 0 bis 30 %, bevorzugt 5 bis 20 %, besonders bevorzugt 10 bis 20 %. 5 bis 30 %, bevorzugt 10 bis 20 % bezogen auf Strom (1) werden als Leichtsiederausschleusung (11) abgezogen. Der Rein-HPN-Abzug (13) beträgt 70 bis 95 Gew.-%, bevorzugt 75 bis 90 Gew.-% bezogen auf Strom (1).

Die Erfindung betrifft auch eine Vorrichtung zur Durchführung des erfindungsgemäßen Verfahrens, umfassend
eine erste Trennstufe aus einem Wischfilmverdampfer (3) und einem Wärmeübertrager (6) und
eine zweite Trennstufe aus einer Rektifizierkolonne (8), zwei Wärmeübertragern (10, 14) und einem zweiten Verdampfer (17)
mit einer Zuführung (1) zum Wischfilmverdampfer (3), einer Zuführung (2) von Fließmittel zum Wischfilmverdampfer (3), einem Auslaß (4) aus dem Wischfilmverdampfer (3), einer Zuführung (5) vom Dampfauslaß des Wischfilmverdampfers (3) zum Wärmeübertrager (6), einer Zuführung (7) vom Auslaß des Wärmeübertragers (6) zur Kolonne (8), einer Zuführung (9) vom Kopf der Kolonne zum Wärmeübertrager (10), einem Abzug (11) aus dem Wärmeübertrager (10), einem Rücklauf (12) vom Wärmeübertrager (10) zum Kopf der Kolonne (8), einem Seitenabzug (13) aus der Kolonne (8) zum Wärmeübertrager (14), einem Auslaß (15) aus dem Wärmeübertrager (14), einem Abzug (16) aus dem Sumpf der Kolonne (8) zum zweiten Verdampfer (17), einer Zuführung (18) vom Sumpf des zweiten Verdampfers (18) zum unteren Bereich der Kolonne (8), einer Zuführung (19) vom Sumpf der Kolonne (8) zum Wischfilmverdampfer (3) der ersten Trennstufe, einer Zuführung (20) vom Ablauf des zweiten Verdampfers (17) zum Wischfilmverdampfer (3) der ersten Trennstufe, einem Auslaß (21) aus dem Sumpf der Kolonne (8), einem Auslaß (22) aus dem Ablauf des zweiten Verdampfers (17).

### Beispiel

In einer wie in Fig. 1 gezeigten Vorrichtung wird ein Strom (1) mit der ungefähren Zusammensetzung von 80 Gew.-% HPN, 18 Gew.-% Leicht-Siedern, 1,8 Gew.-% Hochsiedern und 0,2 Gew.-% anorganischen Salzen eingespeist. Bezogen auf (1) werden 2,0 Gew.-% Polyethylenglykol (P 600) als Fließmittel im Strom (2) einem Wischfilmverdampfer mit beweglichen Wischerelementen (3) zugeführt. Der Wischfilmverdampfer wird bei einem Druck von etwa 6 bis 8 mbar und einer Manteltemperatur von 180°C betrieben. Am Verdampferablauf werden Polyethylenglykol, Hochsieder und anorganische Salze in einer Menge von insgesamt ca. 4 %, bezogen auf Strom (1), als Sumpfstrom (4) ausgetragen. Der verdampfte Produktstrom wird im Wärmeübertrager (6) bei einer Temperatur von 50°C kondensiert und der Rektifizierkolonne (8) zugeführt. Die Kolonne weist bei einem Kopfdruck von 6 bis 8 mbar eine Sumpftemperatur von etwa 160°C, eine Temperatur von 146 bis 155°C am Seitenabzug, eine Temperatur von 100 bis 105°C an der Kondensateinspeisung und eine Temperatur von 95 bis 97°C am Kopf auf. Bei einem Rücklaufverhältnis von (12)/(11) = 15 werden 17 %, bezogen auf Strom (1), als Kopfabzug (11) ausgetragen. Am Seitenabzug wird HPN-Reinware mit ca. 80%, bezogen auf Strom (1), ausgetragen und in einem Wärmeübertrager (14) bei einer Temperatur von 50 bis 60°C kondensiert. Der Verdampfer (17) der zweiten Trennstufe ist als Fallfilmverdampfer ausgeführt. Er wird bei einer Temperatur von 170 bis 180°C betrieben. Der ausgeschleuste Anteil (19) des Kolonnensumpfes beträgt ca. 10%, bezogen auf Strom (1), und wird in den Wischfilmverdampfer (3) der ersten Trennstufe zurückgeführt. Am Seitenabzug wird Reinware mit einem HPN-Gehalt von mehr als 98%, einer Hazen-Farbzahl von weniger als 10 APHA, einem Wassergehalt von weniger als 0,3% und einer Säurezahl von weniger als 5 erhalten. Die Abzüge (20), (21) und (22) sind nicht in Betrieb.

Das erfindungsgemäße Verfahren ermöglicht somit die Reinigung von HPN unter schonenden Bedingungen, so daß ein hoher Anteil an sehr reinem HPN gewonnen werden kann.

## Patentansprüche

1. Verfahren zur Gewinnung von Hydroxypivalinsäureneopentylglykolester (HPN) aus einem HPN, leichter- und höhersiedende Produkte und anorganische Salze enthaltenden Gemisch,
**dadurch gekennzeichnet, daß** das Gemisch in einer ersten Trennstufe aus einem Wischfilmverdampfer und einem Wärmeübertrager
- dem Wischfilmverdampfer zugeführt wird,
- in dem als Sumpf ein Strom aus höhersiedenden Produkten und anorganischen Salzen ausgetragen wird
- und in dem über Kopf ein dampfförmiger Strom aus HPN, leichter- und höhersiedenden Produkten ausgetragen, dem Wärmeübertrager zugeführt und in ihm kondensiert wird, und der erhaltene Kondensatstrom
in einer zweiten Trennstufe aus einer Rektifizierkolonne zur Trennung von HPN, leichter- und höhersiedenden Produkten, einem zweiten Verdampfer und Wärmeübertragern
- der Kolonne zugeführt wird,
- in der über Kopf ein dampfförmiger Strom aus leichtersiedenden Produkten ausgetragen und in einem Wärmeübertrager kondensiert wird,
- das erhaltene Kondensat gegebenenfalls teilweise als Rücklauf auf die Rektifizierkolonne zurückgeführt und teilweise als Leichtsiederausschleusung abgezogen wird,
- in einem Seitenabzug ein dampfförmiger Strom aus HPN ausgetragen und in einem Wärmeübertrager kondensiert wird
- und als Sumpf ein Strom aus HPN und höhersiedenden Produkten ausgetragen wird, der zumindest teilweise dem zweiten Verdampfer zugeführt wird, wobei der Verdampferaustrag ganz oder teilweise in den unteren Bereich der Kolonne zurückgeführt wird.

2. Verfahren nach Anspruch 1, **dadurch gekennzeichnet, daß** die Rektifizierkolonne mindestens drei Trennsektionen übereinander aufweist, die mit druckverlustarmen Einbauten ausgerüstet sein können, wobei der Seitenabzug zwischen der ersten und zweiten Trennsektion angeordnet ist, und der Kondensatstrom aus der ersten Trennstufe zwischen der zweiten und dritten Trennsektion der Kolonne zugeführt wird.

3. Verfahren nach Anspruch 1 oder 2, **dadurch gekennzeichnet, daß** aus dem Sumpf der Rektifizierkolonne ein Strom in den Wischfilmverdampfer der ersten Trennstufe zurückgeführt und/oder ausgeschleust wird.

4. Verfahren nach einem der Ansprüche 1 bis 3, **dadurch gekennzeichnet, daß** aus dem Flüssigkeitsablauf des Verdampfers der zweiten Trennstufe ein Strom in den Wischfilmverdampfer der ersten Trennstufe zurückgeführt und/oder ausgeschleust wird.

5. Verfahren nach einem der Ansprüche 1 bis 4, **dadurch gekennzeichnet, daß** der in der zweiten Trennstufe über Kopf ausgetragene und kondensierte Strom aus leichtersiedende Produkten zumindest teilweise in den Kopf der Kolonne zurückgeführt wird.

6. Verfahren nach einem der Ansprüche 1 bis 5, **dadurch gekennzeichnet, daß** der Verdampfer der zweiten Trennstufe ein Fallfilmverdampfer oder ein Dünnschichtverdampfer ist.

7. Verfahren nach einem der Ansprüche 1 bis 6, **dadurch gekennzeichnet, daß** das eingesetzte Gemisch zusätzlich ein hochsiedendes Fließmittel enthält.

8. Verfahren nach einem der Ansprüche 1 bis 7, **dadurch gekennzeichnet, daß** in der ersten Trennstufe eines oder mehrere der nachstehenden Merkmale verwirklicht sind:
- Druck im Wischfilmverdampfer 2 bis 15 mbar
- Manteltemperatur des Wischfilmverdampfers 140 bis 200°C
- Druck im Wärmeübertrager 2 bis 15 mbar
- Temperatur im Wärmeübertrager 30 bis 130°C.

9. Verfahren nach einem der Ansprüche 1 bis 8, **dadurch gekennzeichnet, daß** in der zweiten Trennstufe eines oder mehrere der nachstehenden Merkmale verwirklicht sind:
- Temperatur des der Kolonne aus der ersten Trennstufe zugeführten Kondensats 30 bis 130°C
- Temperatur im Sumpf der Kolonne 150 bis 180°C
- Temperatur zwischen der ersten und zweiten Trennsektion 150 bis 170°C
- Temperatur zwischen der zweiten und dritten Trennsektion 150 bis 170°C
- Temperatur am Kolonnenkopf 90 bis 100°C
- Temperatur des Kondensats von HPN am Seitenabzug 30 bis 130°C
- Temperatur im zweiten Verdampfer 140 bis 200°C
- am Seitenabzug gewonnenes HPN hat einen HPN-Gehalt von mehr 98 Gew.-%, eine Hazen-Farbzahl von weniger als 10 APHA, einen Wassergehalt von weniger als 0,3 Gew.-% und eine Säurezahl von weniger als 5.

10. Vorrichtung zur Durchführung des Verfahrens nach einem der Ansprüche 1 bis 9, umfassend
eine erste Trennstufe aus einem Wischfilmverdampfer (3) und einem Wärmeübertrager (6) und
eine zweite Trennstufe aus einer Rektifizierkolonne (8), zwei Wärmeübertragern (10, 14) und einem zweiten Verdampfer (17),
mit einer Zuführung (1) von Roh-HPN zum Wischfilmverdampfer (3), einer Zuführung (2) für Fließmittel zum Wischfilmverdampfer (3), einem Auslaß (4) aus dem Wischfilmverdampfer (3), einer Zuführung (5) vom Dampfauslaß des Wischfilmverdampfers (3) zum Wärmeübertrager (6), einer Zuführung (7) vom Auslaß des Wärmeübertragers (6) zur Kolonne (8), einer Zuführung (9) vom Kopf der Kolonne zum Wärmeübertrager (10), einem Abzug (11) aus dem Wärmeübertrager (10), einem Rücklauf (12) vom Wärmeübertrager (10) zum Kopf der Kolonne (8), einem Seitenabzug (13) aus der Kolonne (8) zum Wärmeübertrager (14), einem Auslaß (15) aus dem Wärmeübertrager (14), einem Abzug (16) aus dem Sumpf der Kolonne (8) zum zweiten Verdampfer (17), einer Zuführung (18) vom Sumpf des zweiten Verdampfers (17) zum unteren Bereich der Kolonne (8), einer Zuführung (19) vom Sumpf der Kolonne (8) zum Wischfilmverdampfer (3) der ersten Trennstufe, einer Zuführung (20) vom Ablauf des zweiten Verdampfers (17) zum Wischfilmverdampfer (3) der ersten Trennstufe, einem Auslaß (21) aus dem Sumpf der Kolonne (8), einem Auslaß (22) aus dem Ablauf des zweiten Verdampfers (17).

## Claims

1. A process for isolating neopentyl glycol hydroxypivalate (NGH) from a mixture containing NGH, lower- and higher-boiling products and inorganic salts,
which comprises the mixture being, in a first separation stage consisting of a wiped-film evaporator and a heat exchanger,
- fed to the wiped-film evaporator,
- in which a stream of higher-boiling products and inorganic salts is discharged as bottom product
- and in which a distillate stream consisting of NGH, lower- and higher-boiling products is discharged and fed to the heat exchanger and condensed therein, and the resulting condensate stream being,
in a second separation stage consisting of a rectification column for separating NGH, lower- and higher-boiling products, a second evaporator and heat exchangers,
- fed to the column,
- in which a distillate stream consisting of lower-boiling products is discharged and condensed in a heat exchanger,
- the resulting condensate is, where appropriate, partly recycled to the rectification column and partly taken off as low-boiler stream,
- a distillate sidestream of NGH is discharged and condensed in a heat exchanger
- a bottom stream of NGH and higher-boiling products is discharged and at least partly fed to the second evaporator, with all or part of the discharge from the evaporator being recycled to the lower region of the column.

2. A process as claimed in claim 1, wherein the rectification column has at least three separation sections one above the other, which can be equipped with internals with a low pressure drop, with the sidestream being taken off between the first and second separation sections, and the condensate stream from the first separation section being fed to the column between the second and third separation sections.

3. A process as claimed in claim 1 or 2, wherein a stream of bottom product from the rectification column is recycled to the wiped-film evaporator in the first separation stage and/or is discharged.

4. A process as claimed in any of claims 1 to 3, wherein a liquid stream from the evaporator in the second separation stage is recycled to the wiped-film evaporator in the first separation stage and/or is discharged.

5. A process as claimed in any of claims 1 to 4, wherein the condensed distillate of lower-boiling products in the second separation stage is at least partly recycled to the top of the column.

6. A process as claimed in any of claims 1 to 5, wherein the evaporator in the second separation stage is a falling film evaporator or a thin-film evaporator.

7. A process as claimed in any of claims 1 to 6, wherein the mixture employed additionally comprises a high-boiling fluidifier.

8. A process as claimed in any of claims 1 to 7, wherein one or more of the following features are implemented in the first separation stage:
- pressure in the wiped-film evaporator 2 to 15 mbar
- jacket temperature of the wiped-film evaporator 140 to 200°C
- pressure in the heat exchanger 2 to 15 mbar
- temperature in the heat exchanger 30 to 130°C.

9. A process as claimed in any of claims 1 to 8, wherein one or more of the following features are implemented in the second separation stage:
- temperature of the condensate fed to the column from the first separation stage 30 to 130°C
- bottom temperature of the column 150 to 180°C
- temperature between the first and second separation sections 150 to 170°C
- temperature between the second and third separation sections 150 to 170°C
- temperature at the top of the column 90 to 100°C
- temperature of the sidestream NGH condensate 30 to 130°C
- temperature in the second evaporator 140 to 200°C
- the NGH sidestream has an NGH content of more than 98% by weight, a Hazen color number of less than 10 APHA, a water content of less than 0.3% by weight and an acid number of less than 5.

10. An apparatus for carrying out the process as claimed in any of claims 1 to 9, comprising
a first separation stage consisting of a wiped-film evaporator (3) and a heat exchanger (6) and
a second separation stage consisting of a rectification column (8), two heat exchangers (10, 14) and a second evaporator (17)
with a feed (1) of crude NGH to the wiped-film evaporator (3), a feed (2) for fluidifier to the wiped-film evaporator (3), an outlet (4) from the wiped-film evaporator (3), a feed (5) for distillate from the wiped-film evaporator (3) to the heat exchanger (6), a feed (7) from the heat exchanger (6) to the column (8), a feed (9) for distillate from the column to the heat exchanger (10), a discharge (11) from the heat exchanger (10), a feed (12) from the heat exchanger (10) to the top of column (8), a side discharge (13) from column (8) to the heat exchanger (14), an outlet (15) from the heat exchanger (14), a discharge (16) from the bottom of column (8) to the second evaporator (17), a feed (18) from the bottom of the second evaporator (17) to the lower part of column (8), a feed (19) from the bottom of column (8) to the wiped-film evaporator (3) in the first separation stage, a feed (20) from the second evaporator (17) to the wiped-film evaporator (3) in the first separation stage, an outlet (21) from the bottom of column (8), an outlet (22) from the second evaporator (17).

## Revendications

1. Procédé de purification de néopentylglycolester de l'acide hydroxypivalique (HPN) à partir d'un mélange contenant de l'HPN, des produits légers et lourds et des sels inorganiques, **caractérisé en ce que**, dans une première étape de séparation constituée d'un évaporateur pelliculaire à balayage et d'un échangeur de chaleur, le mélange
- est envoyé dans l'évaporateur pelliculaire à balayage,
- où l'on soutire en fraction de fond un courant de produits lourds et des sels inorganiques
- et où l'on soutire en fraction de tête un courant de vapeurs de HPN, de produits légers et lourds, que l'on envoie à l'échangeur de chaleur où il se condense, et le courant de condensat obtenu est,
dans une deuxième étape de séparation constituée d'une colonne de rectification pour la séparation du HPN et des produits légers et lourds, d'un deuxième évaporateur et d'un échangeur de chaleur,
- envoyé dans la colonne,
- où un courant de vapeurs de produits légers est soutiré en tête et condensé dans un échangeur de chaleur,
- le condensat obtenu est éventuellement renvoyé en partie dans la colonne de rectification en tant que reflux et en partie soutiré comme évacuation de produits légers,
- un courant de vapeurs de HPN est soutiré par un soutirage latéral et condensé dans un échangeur de chaleur,
- et l'on soutire en fraction de fond un courant de HPN et de produits lourds, qui est envoyé au moins en partie au deuxième évaporateur, la sortie de l'évaporateur étant totalement ou partiellement renvoyée dans la partie inférieure de la colonne.

2. Procédé selon la revendication 1, **caractérisé en ce que** la colonne de rectification présente au moins trois zones de séparation superposées, qui peuvent être équipées d'éléments à faible perte de charge, le soutirage latéral étant placé entre les première et deuxième sections de séparation, et le courant de condensat sortant de la première étape de séparation étant amené entre les deuxième et troisième sections de séparation de la colonne.

3. Procédé selon la revendication 1 ou 2, **caractérisé en ce qu'**un courant provenant du fond de la colonne de rectification est renvoyé dans l'évaporateur pelliculaire à balayage et/ou évacué.

4. Procédé selon l'une des revendications 1 à 3, **caractérisé en ce qu'**un courant soutiré de la sortie de liquide de l'évaporateur de la deuxième étape de séparation est renvoyé dans l'évaporateur pelliculaire à balayage et/ou évacué.

5. Procédé selon l'une des revendications 1 à 4, **caractérisé en ce que**, dans la deuxième étape de séparation, un courant de produits légers soutiré en tête et condensé est tout au moins partiellement renvoyé en tête de la colonne.

6. Procédé selon l'une des revendications 1 à 5, **caractérisé en ce que** l'évaporateur de la deuxième étape de séparation est un évaporateur à film descendant ou un évaporateur à couche mince.

7. Procédé selon l'une des revendications 1 à 6, **caractérisé en ce que** le mélange mis en oeuvre contient en outre un agent solvant à haut point d'ébullition.

8. Procédé selon l'une des revendications 1 à 7, **caractérisé en ce que**, dans la première étape de séparation, sont réalisées une ou plusieurs des caractéristiques suivantes:
- la pression dans l'évaporateur pelliculaire à balayage est de 2 à 15 mbar
- la température du manteau de l'évaporateur pelliculaire à balayage est de 140 à 200°C
- la pression dans l'échangeur de chaleur est de 2 à 15 mbar
- la température dans l'échangeur de chaleur est de 30 à 130°C.

9. Procédé selon l'une des revendications 1 à 8, **caractérisé en ce que**, dans la deuxième étape de séparation, sont réalisées une ou plusieurs des caractéristiques suivantes:
- la température du condensat amené à la colonne en provenance de la première étape de séparation est de 30 à 130°C
- la température du fond de colonne est de 150 à 180°C
- la température entre les première et deuxième sections de séparation est de 150 à 170°C
- la température entre les deuxième et troisième sections de séparation est de 150 à 170°C
- la température en tête de colonne est de 90 à 100°C
- la température du condensat de HPN au soutirage latéral est de 30 à 130°C
- la température dans le deuxième évaporateur est de 140 à 200°C
- le HPN récupéré au soutirage latéral présente une teneur en HPN de plus de 98% en poids, un indice de trouble de moins de 10 APHA, une teneur en eau de moins de 0,3% en poids et un indice d'acidité inférieur à 5.

10. Dispositif pour l'exécution du procédé selon l'une des revendications 1 à 9, comprenant:
une première étape de séparation constituée d'un évaporateur pelliculaire à balayage (3) et d'un échangeur de chaleur (6) et
une deuxième étape constituée d'une colonne de rectification (8), deux échangeurs de chaleur (10,14) et un deuxième évaporateur (17),
avec une alimentation (1) de HPN brut vers l'évaporateur pelliculaire à balayage (3), une alimentation (2) d'agent solvant vers l'évaporateur pelliculaire à balayage (3), une sortie (4) de l'évaporateur pelliculaire à balayage (3), une amenée (5) de sortie de vapeurs de l'évaporateur pelliculaire à balayage (3) vers l'échangeur de chaleur (6), une amenée (7) de la sortie de l'échangeur de chaleur (6) vers la colonne (8), une amenée (9) de la tête de la colonne vers l'échangeur de chaleur (10), une sortie (11) de l'échangeur de chaleur (10), un reflux (12) de l'échangeur de chaleur (10) dans la tête de la colonne (8), un soutirage latéral (13) de la colonne (8) vers l'échangeur de chaleur (14), une sortie (15) de l'échangeur de chaleur (14), un soutirage (16) du fond de la colonne (8) vers le deuxième évaporateur (17), une amenée (18 ) du fond du deuxième évaporateur (17) au bas de la colonne (8), une amenée (19) du fond de la colonne (8) vers l'évaporateur pelliculaire à balayage (3) de la première étape de séparation, une amenée (20) de la sortie du deuxième évaporateur (17) vers l'évaporateur pelliculaire à balayage (3) de la première étape de séparation, une sortie (21) du fond de la colonne (8), une sortie (22) de la décharge du deuxième évaporateur (17).
